# EUROPEAN PATENT APPLICATION

(11) **EP 2 485 048 A1**
(43) Date of publication of application: **08.08.2012**
(21) Application number: 12153726.0
(22) Date of filing: 02.02.2012
(51) Int. Cl.: G01N 33/49, G01N 27/327

(54) **Analysis device for blood sugar level with test element insertion guiding means**

(30) Priority: 02.02.2011 JP 2011020427; 12.12.2011 JP 2011270850
(71) Applicant: Arkray, Inc., Kyoto-shi, Kyoto 601-8045 (JP)
(72) Inventor: Nakagawa, Takashi, Kyoto-shi, Kyoto 6020008 (JP)
(74) Representative: Piésold, Alexander James

(57) **Abstract**

The analysis device (101; 102; 103; 104; 105; 106; 107; 108) includes a main body (200), an insertion slot (210) into which a sensor piece (500) is inserted in a manner such that the sensor piece sticks out from the main body in a measurement operation, and a pair of projections (220; 320; 420) spaced apart from each other in a y-direction, with the insertion slot present between the projections. Each of the projections protrudes outwardly in an x-direction and has a height overlapping the insertion slot in a z-direction.

## Description

### BACKGROUND OF THE INVENTION

### 1. Field of the Invention:

The present invention relates to an analysis device to be used, for example, for measuring a blood sugar level.

### 2. Description of the Related Art:

Analysis devices to be used, for example, by diabetes patients for measuring their blood sugar level have thus far been proposed. Fig. 18 depicts an example of the conventional analysis device (see JP-A1-01/061341). The analysis device 900 shown in the figure includes a main body 901 with an insertion slot 902. The insertion slot 902 serves to receive a sensor piece 910. For measurement with the analysis device 900, the sensor piece 910 is disposed so as to stick out from the insertion slot 902 in an x-direction and a droplet of specimen (blood) is dispensed on the sensor piece 910. A V-shaped guide 903 is attached to the main body 901. The guide 903 serves to guide the sensor piece 910 toward the insertion slot 902.

Diabetes patients are prone to suffer amblyopia, originating from diabetic retinopathy. Generally, patients suffering amblyopia can barely visually recognize the detailed shape of the analysis device 900, although they can manage to recognize the presence of the analysis device 900. Accordingly, it is difficult for such patients to accurately recognize the position of the insertion slot 902, when they try to search for the guide 903 with a hand holding the analysis device 900 and to insert the sensor piece 910 with the other hand.

### SUMMARY OF THE INVENTION

The present invention has been proposed under the foregoing situation, and an object thereof is to provide an analysis device with which users can properly perform a measurement without depending on their vision.

According to the present invention, there is provided an analysis device that comprises: a main body; a sensor piece slot that supports a sensor piece in a manner such that the sensor piece sticks out from the main body; and a pair of projections spaced apart from each other in a width direction perpendicular to a sticking direction of the sensor piece, with the sensor piece slot present between the projections. Each of the projections protrudes outwardly in the sticking direction of the sensor piece and has a height overlapping the sensor piece slot in a height direction perpendicular to both the sticking direction and the width direction.

In a preferred embodiment of the present invention, the sensor piece slot may be configured for insertion of the sensor piece from an outside of the analysis device.

In a preferred embodiment of the present invention, the analysis device may further include a guide surface adjacent to the sensor piece slot and extending from the sensor piece slot in the sticking direction.

In a preferred embodiment of the present invention, the sensor piece slot, together with the guide surface, may be at a position offset downwardly in the height direction, i.e., in a direction proceeding from the slot toward the guide surface in the height direction.

In a preferred embodiment of the present invention, the analysis device may further include at least a pair of sloped surfaces adjacent to the sensor piece slot, with the sensor piece slot present between the sloped surfaces, where the sloped surfaces are arranged to become further from each other as proceeding away from the sensor piece slot in the sticking direction.

In a preferred embodiment of the present invention, each of the projections may include an elevated portion protruding in the height direction.

In a preferred embodiment of the present invention, the elevated portion may be located outwardly of the sensor piece slot in the sticking direction.

In a preferred embodiment of the present invention, the projections may be movable relative to each other in the width direction.

In a preferred embodiment of the present invention, the projections may move symmetrically with respect to the center of the sensor piece slot.

In a preferred embodiment of the present invention, the analysis device may further include a resistance applier that applies a resisting force to the sensor piece inserted through the sensor piece slot at a resistance applying position, wherein, when the sensor piece is in a measurement position, the distance between a leading end of the respective projections in the sticking direction and a leading end of the sensor piece in the sticking direction may be shorter than the distance between an opposite end of the sensor piece and the resistance applying position.

In a preferred embodiment of the present invention, the resistance applier may include an electrode coming into contact with the sensor piece or a detection lever for detecting the presence of the sensor piece.

In a preferred embodiment of the present invention, the projections may be formed of a material softer than a material of the main body.

In a preferred embodiment of the present invention, the analysis device may further include an attachment removably attachable to the main body, where the projections may be formed on the attachment.

In a preferred embodiment of the present invention, the attachment may be provided with an audible guidance function.

With the above-noted arrangements, for example, the user can hold the analysis device by sandwiching the projections between the thumb and the index finger in the height direction. In this manner, a space is defined by the user's thumb, index finger and the projections, and in this space the sensor piece slot is disposed at an inner position in the sticking direction. Keeping such a configuration in mind, the user can feel for the sensor piece slot, and manage to insert the sensor piece into the slot without visual recognition of the sensor piece slot. Further, the user can readily apply, without visual recognition again, a droplet of specimen, such as blood, to the sensor piece supported by the sensor piece slot. Thus, a proper measurement operation can be performed without relying on visual sense.

### BRIEF DESCRIPTION OF THE DRAWINGS

Certain preferred embodiments of the present invention will now be described in great detail by way of example only and with reference to the accompanying drawings in which:
Fig. 1 is a perspective view showing an analysis device according to a first embodiment of the present invention;
Fig. 2 is a plan view showing the analysis device shown in Fig. 1;
Fig. 3 is a front view showing the analysis device shown in Fig. 1;
Fig. 4 is a partial cross-sectional view taken along a line IV-IV in Fig. 3;
Fig. 5 is a partial cross-sectional view showing a sensor piece located at a resistance applying position;
Fig. 6 is a partial cross-sectional view showing the sensor piece located at a measurement position;
Fig. 7 is a partial cross-sectional view of an analysis device according to a second embodiment of the present invention;
Fig. 8 is a partial cross-sectional view showing the sensor piece located at the resistance applying position;
Fig. 9 is a partial cross-sectional view showing the sensor piece located at the measurement position;
Fig. 10 is a perspective view showing an analysis device according to a third embodiment of the present invention;
Fig. 11 is a plan view showing an analysis device according to a fourth embodiment of the present invention;
Fig. 12 is an exploded perspective view showing an analysis device according to a fifth embodiment of the present invention;
Fig. 13 is a perspective view showing the analysis device shown in Fig. 9;
Fig. 14 is a bottom view showing an analysis device according to a sixth embodiment of the present invention;
Fig. 15 is an exploded perspective view showing an analysis device according to a seventh embodiment of the present invention;
Fig. 16 is a perspective view showing the analysis device shown in Fig. 15;
Fig. 17 is a perspective view showing an analysis device according to an eighth embodiment of the present invention; and

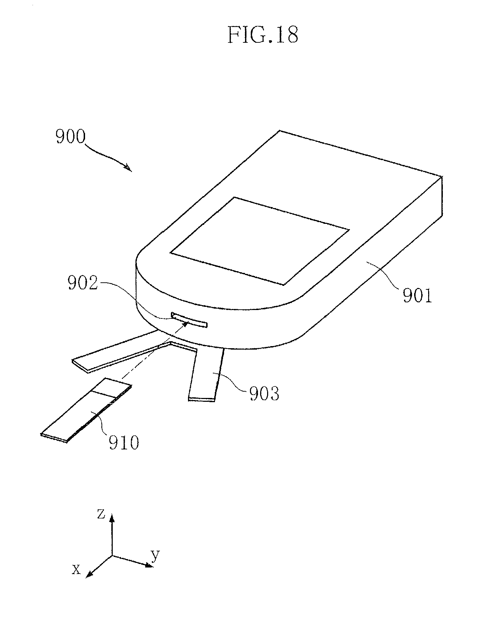

Fig. 18 is a perspective view showing a conventional analysis device.

### DETAILED DESCRIPTION OF THE PREFERRED EMBODIMENTS

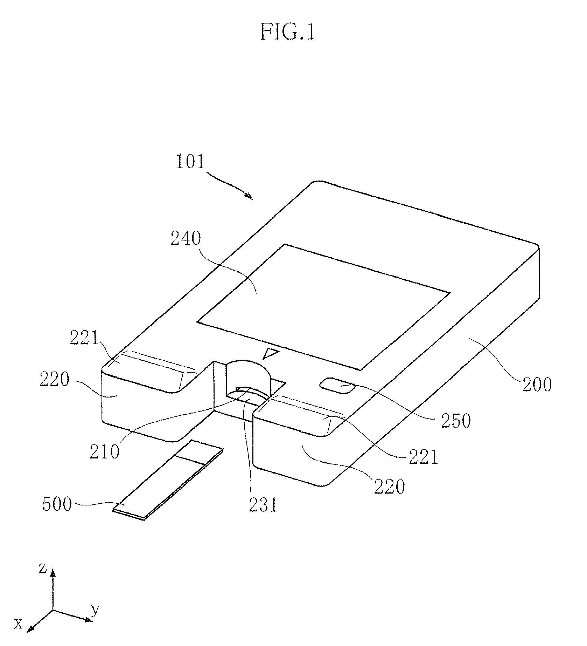

Figs. 1-6 depict an analysis device according to a first embodiment of the present invention. The analysis device 101 according to this embodiment includes a main body 200, and the main body 200 is provided with an insertion slot 210, a pair of projections 220, a guide surface 231, a display panel 240, and a power button 250. The analysis device 101 is used, for example, by diabetes patients for measuring their blood sugar level, and a sensor piece 500 is attached to the analysis device 101 for the measurement operation.

The main body 200 is a box-shaped component formed of a resin, for example. The insertion slot 210 is formed in an end face of the main body 200 in an x-direction (sticking direction according to the present invention), so as to have a slender rectangular cross-sectional shape with the longitudinal sides extending in a y-direction (width direction according to the present invention). A strip-shaped sensor piece 500 is inserted in the insertion slot 210. During the measurement by the analysis device 101, the sensor piece 500 is disposed so as to stick out from the insertion slot 210. Thus, the insertion slot 210 exemplifies the sensor piece slot according to the present invention. Here, the sensor piece 500 may be placed in advance inside the main body 200, instead of being inserted from outside as in this embodiment, and made to stick out from the sensor piece slot according to the present invention.

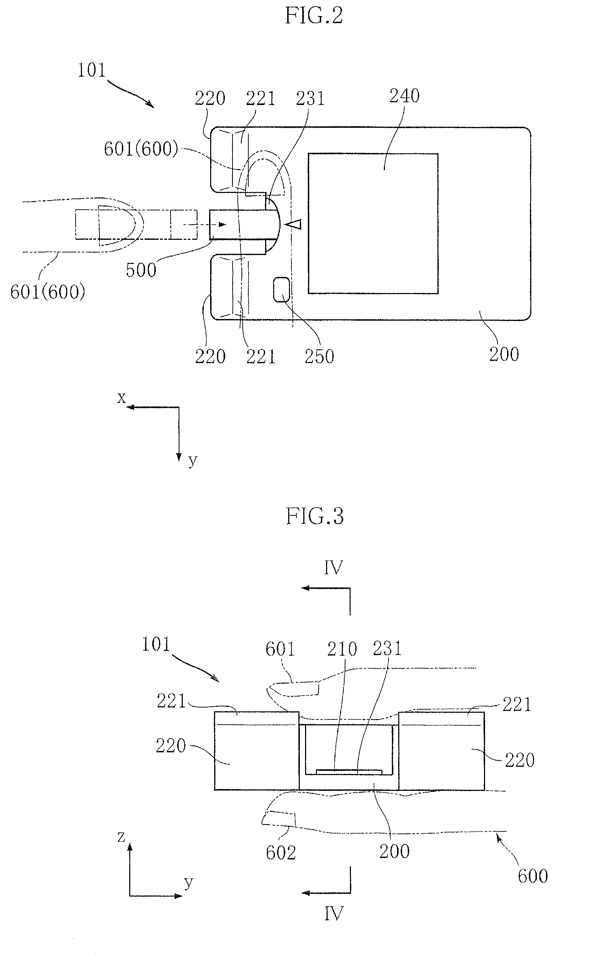

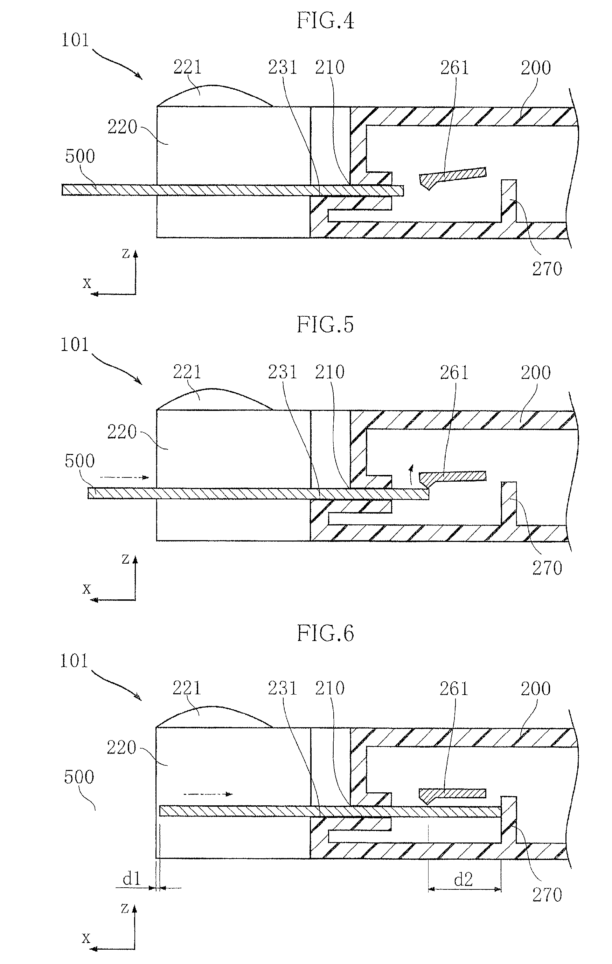

The guide surface 231 is formed so as to outwardly extend in the x-direction from a position adjacent to the lower edge of the insertion slot 210 in a z-direction (height direction according to the present invention). An upper region of the insertion slot 210 in the z-direction constitutes a surface extending in the z-direction. Accordingly, the guide surface 231 located at the lower position of the insertion slot 201 in the z-direction protrudes in the x-direction. As shown in Figs. 3 and 4, the insertion slot 210 is at a position that is offset downwardly in the z-direction, in other words, offset in a direction proceeding from the slot toward the guide surface 231 so as to be closer to the lower surface of the main body 200.

As shown in Figs. 1 to 3, the projections 220 have a predetermined in-between clearance, in other words, are spaced apart from each other in the y-direction, with the insertion slot 210 present between them. The projections 220 each protrude outwardly in the x-direction with respect to the insertion slot 210. The projections 220 have generally the same size in the z-direction as the remaining portions of the main body 200. Thus, the projections 220 have a height that overlaps the insertion slot 210 in the z-direction. In this embodiment, the projections 220 are of a rectangular block shape as a whole. The projections 220 each include an elevated portion 221. The elevated portion 221 is formed on an upper surface of the projection 220 in the z-direction, in a shape having a low, triangular cross-section taken in a zx-plane as shown in Figs. 4-6. The elevated portion 221 is located outwardly of the insertion slot 210 in the x-direction. The projections 220 may be formed integrally with the remaining portions of the main body 200. Alternatively, the projections 220 may be prepared separately from the remaining portions of the main body 200 by using a different material, and configured to be coupled to the main body 200. In this case, the projections 220 may be formed of a relatively soft material such as a silicone resin, and the remaining portions of the main body 200 other than the projections 220 may be formed of a conventional hard plastic. Further, the portions of the main body 200 other than the projection 220 may be provided with smooth surfaces, whereas the projections 220 may be provided with slightly uneven surfaces. Providing those surfaces with different textures enables a weak-sighted user to readily feel for the projections 220.

As shown in Fig. 1, the display panel 240 is provided on the upper surface of the main body 200 in the z-direction. The display panel 240 may be an LCD for example, and serves to display the status of the analysis device 101, a measurement result, and so forth. The power button 250 is located in the vicinity of the display panel 240, to be used to turn on and off the analysis device 101. Here, the analysis device 101 may be turned on by inserting the sensor piece 500 and turned off by removing the sensor piece 500. In this case, the power button 250 is unnecessary.

An operation of the analysis device 101 will now be described hereunder.

To measure for example the blood sugar level with the analysis device 101, the sensor piece 500 is attached to the analysis device 101 as shown in Fig. 1. The sensor piece 500 includes a portion where a specimen such as blood is to be applied, an electrode portion for electrical connection to the analysis device 101, and so forth, formed in advance. First, as shown in Figs. 2 and 3, the user holds the analysis device 101 with a hand 600. More specifically, the user holds the projections 220 between the thumb 601 and the index finger 602 in the z-direction. Preferably, the thumb 601 may be placed on the inner side in the x-direction, of the elevated portion 221 formed on the projection 220. Holding thus the analysis device 101 brings the insertion slot 210 to a position between the thumb 601 and the index finger 602 in the z-direction. Then the user picks up the sensor piece 500 with the other hand 600, and passes the sensor piece 500 through the space defined by the projections 220, the thumb 601, and the index finger 602, so as to insert the sensor piece 500 into the insertion slot 210.

As shown in Fig. 4, an electrode 261 and a stopper 270 are provided inside the insertion slot 210. The electrode 261 serves for electrical connection with the electrode of the sensor piece 500, and is rotatable about an axis extending in the y-direction. The electrode 261 has elasticity that allows the left end portion thereof to be pressed downward in the state shown in Fig. 4. The electrode 261 is an example of the resistance applier according to the present invention. The stopper 270 is located inwardly of the electrode 261 in the x-direction, and serves to delimit the travel distance of the sensor piece 500 to thereby set the sensor piece 500 at the measurement position.

Upon inserting the sensor piece 500 deeper inside in the x-direction, the right end portion of the sensor piece 500 contacts the electrode 261 as shown in Fig. 5. To insert the sensor piece 500 still deeper inside in the x-direction, the sensor piece 500 lifts the electrode 261. The reaction force of the electrode 261 against the lifting action constitutes a resisting force imposed on the sensor piece 500. Such a position of the sensor piece 500 corresponds to the resistance applying position according to the present invention.

Upon inserting the sensor piece 500 even deeper inside, the right end portion of the sensor piece 500 abuts the stopper 270, as shown in Fig. 6. Thus, the sensor piece 500 is unable to proceed any further. The analysis device 101 may be configured so as to notify the user of the detection of this position, by a buzzer or an audible message. Such a position of the sensor piece 500 corresponds to the measurement position according to the present invention. A distance d1 in the x-direction between the left end portion of the sensor piece 500 located at the measurement position and the left end portion of the projection 220 is shorter than a distance d2 between the resistance applying position and the right end portion of the sensor piece when the sensor piece is in the measurement position. It is preferable that the distance d1 is zero.

Thereafter, a predetermined operation for the measurement including spot application of blood to the sensor piece 500 is performed, to thereby measure the blood sugar level.

Hereafter, the advantageous effects of the analysis device 101 will be described.

The configuration according to this embodiment allows the user to hold the projections 220 by using the thumb 601 and the index finger 602 as shown in Figs. 2 and 3. Consequently, a space is defined by the thumb 601, the index finger 602, and the projections 220, and in this space, the insertion slot 210 is to be located at an inner position in the x-direction. Accordingly, the user can feel for the insertion slot 210 so as to insert the sensor piece 500 therein, without visually recognizing the position of the insertion slot 210. Hence, the user can properly perform the measurement without depending on vision.

The elevated portion 221 formed on the projections 220 allows the user to easily recognize the orientation of the analysis device 101 in the z-direction, through the user's sense of touch. In addition, placing the thumb 601 on the inner side of the elevated portion 221 in the x-direction allows the user to detect the approximate position of the insertion slot 210 in the x-direction. Since the guide surface 231 is provided, upon moving the sensor piece 500 downward from an upper position in the z-direction before the sensor piece 500 reaches the insertion slot 210, the sensor piece 500 is received by the guide surface 231. Then upon pressing the sensor piece 500 inward in the x-direction in this state, the sensor piece 500 can be properly inserted in the insertion slot 210. Forming the projections 220 from a relatively soft material facilitates the user to retain the analysis device 101 with the hand 600.

As shown in Fig. 5, the sensor piece 500 inserted through the insertion slot 210 is subjected to the resisting force at the resistance applying position. If the feeling of the resistance induced the user suffering amblyopia to decide that the sensor piece 500 had reached the measurement position, the user would fail to perform a proper measurement. In this embodiment, however, the distance d1 is shorter than the distance d2 as shown in Fig. 6. Accordingly, the sensor piece 500 prominently sticks out from the projections 220, when the sensor piece 500 is at the resistance applying position. The user can easily feel for the projection of the sensor piece 500 so as to promptly recognize that the sensor piece 500 has not yet reached the measurement position, to thereby prevent insufficient insertion of the sensor piece 500. To ensure such an effect, it is preferable that the distance d1 is zero.

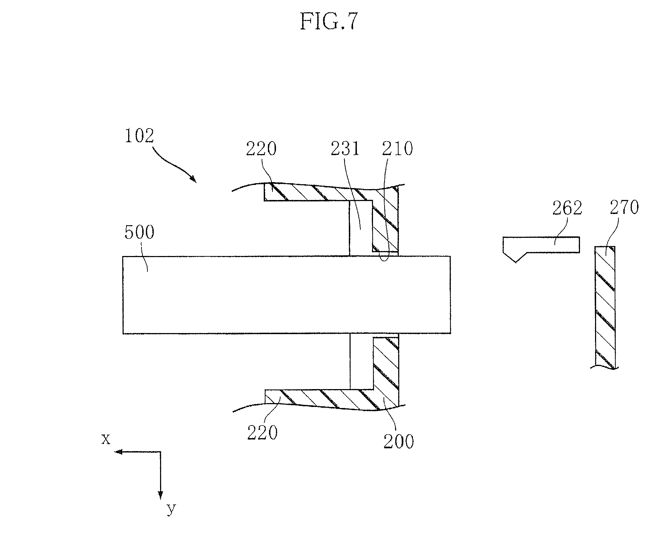

Figs. 7-16 depict other embodiments of the present invention. In these drawings, the constituents same as or similar to those of the first embodiment are given the same numeral.

Figs. 7 to 9 depict an analysis device according to a second embodiment of the present invention. The analysis device 102 according to this embodiment includes a detection lever 262 that serves as the resistance applier. The detection lever 262 is employed for detecting the insertion of the sensor piece 500, and connected to a sensor which is not shown. The detection lever 262 is rotatable about an axis extending in the z-direction, and has elasticity that allows the left end portion of the detection lever 262 to be pressed downward in Fig. 7, in the state shown therein.

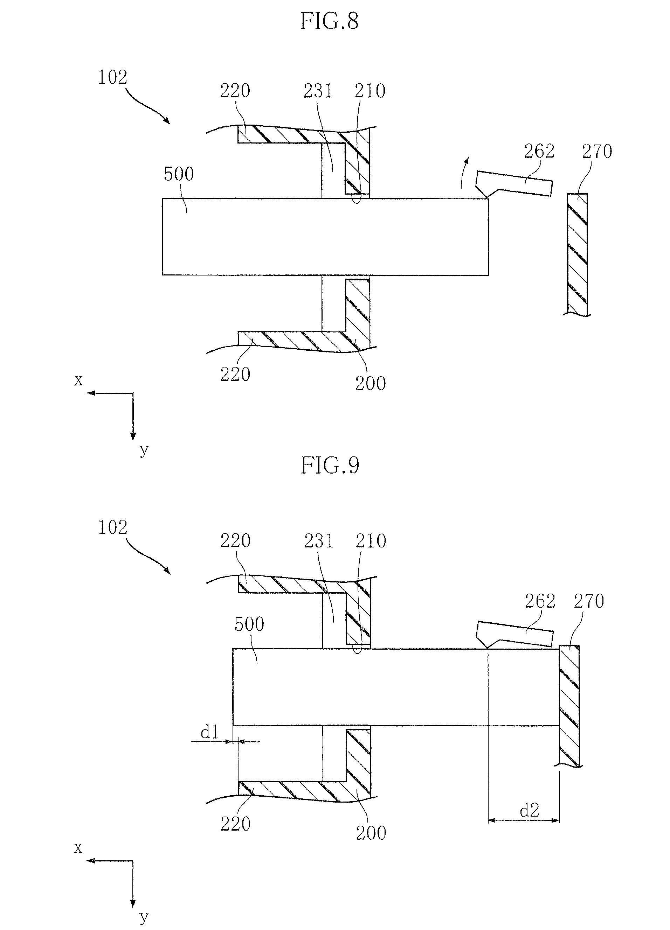

As shown in Fig. 8, upon inserting the sensor piece 500 the right end portion thereof contacts the detection lever 262. Accordingly, the detection lever 262 is made to rotate by the sensor piece 500. At this moment, the sensor piece 500 is subjected to an outward resisting force in the x-direction, generated by the detection lever. The state shown in Fig. 8 represents the resistance applying position in the analysis device 102.

Fig. 9 depicts the measurement position in the analysis device 102. In this embodiment also, the distance d1 is shorter than the distance d2. Such a configuration according to this embodiment can also prevent insufficient insertion of the sensor piece 500. Here, both of the electrode 261 and the detection lever 262 may be provided so as to constitute the resistance applier.

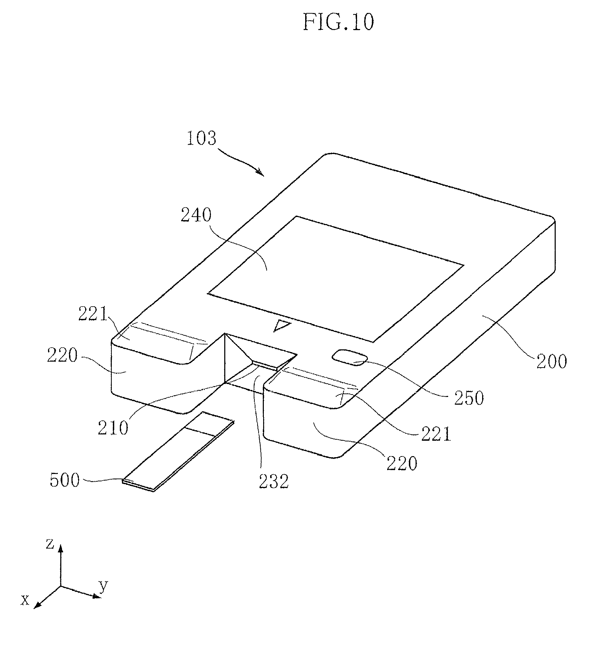

Fig. 10 depicts an analysis device according to a third embodiment of the present invention. The analysis device 103 according to this embodiment is different from the foregoing embodiments in including two pairs of sloped surfaces 232. The two pairs of sloped surfaces 232 are arranged to surround the insertion slot 210. The sloped surfaces 232 in one pair are spaced apart from each other in the z-direction with the insertion slot 210 present therebetween, while the sloped surfaces 232 in the other pair are spaced apart from each other in the y-direction with the insertion slot 210 present therebetween. The two sloped surfaces 232 of the respective pair are arranged to become farther from each other as proceeding outer in the x-direction from the insertion slot 210.

With the configuration according to this embodiment, although the sensor piece 500 is brought to a position deviated from the insertion slot 210 in the y-direction and/or the z-direction, the two pairs of sloped surfaces 232 can smoothly guide the sensor piece 500 to the insertion slot 210.

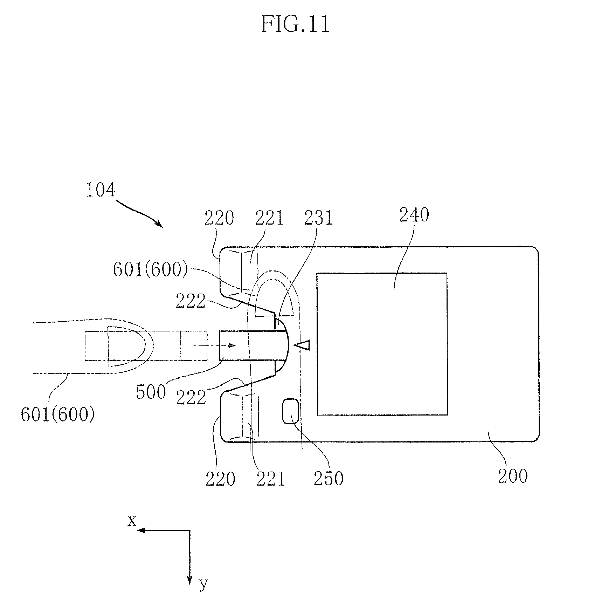

Fig. 11 depicts an analysis device according to a fourth embodiment of the present invention. The analysis device 104 according to this embodiment is different from the foregoing embodiments in including inner walls 222 formed on the projections 220, respectively. Of all the surfaces of the respective projections 220, the inner walls 222 are arranged on a side close to the insertion slot 210. Thus, the analysis device 104 includes a pair of inner walls 222 which are spaced apart from each other in the y-direction, with the insertion slot 210 present therebetween. The inner walls 222 are sloped such that they become farther from each other as proceeding outwardly in the x-direction from the insertion slot 210. With these arrangements, the inner walls 222 can guide the sensor piece 500 to the center therebetween as the sensor piece 500 is being inserted. Thus, the sensor piece 500 can be properly inserted.

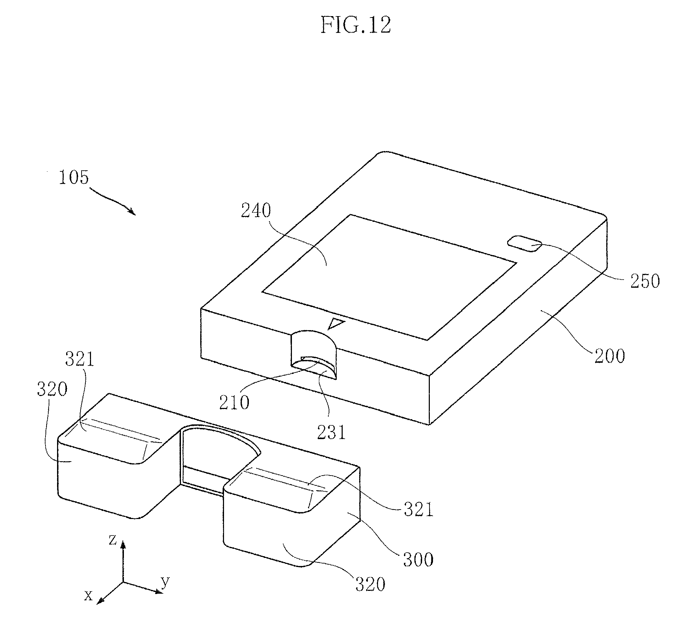

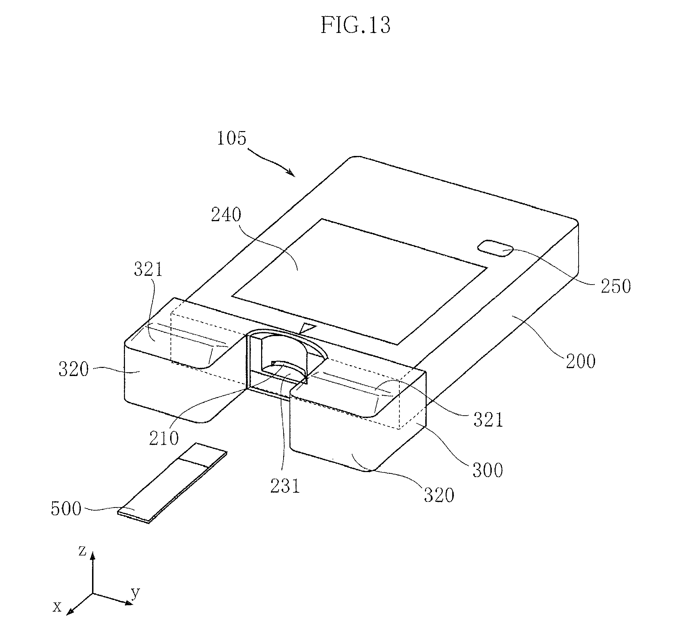

Figs. 12 and 13 depict an analysis device according to a fifth embodiment of the present invention. The analysis device 105 according to this embodiment is different from the foregoing embodiments in including an attachment 300 to be attached to the main body 200. As shown in Fig. 12, the main body 200 does not include the projection according to the present invention. The main body 200 thus configured can fulfill the measurement function, in the case where convenience of use by a user suffering amblyopia may be disregarded. The attachment 300 can be attached to the main body 100, and includes a pair of projections 320. The projections 320 are of a size similar to the projections 220, and includes an elevated portion 321 formed thereon similarly to the elevated portion 221.

As shown in Fig. 13, when the attachment 300 is attached to the main body 200, the projections 320 are spaced apart from each other in the y-direction, with the insertion slot 210 present therebetween. The positional relationship between the insertion slot 210 and the projections 320 is the same as that between the insertion slot 210 and the projections 220 according to the foregoing embodiments.

The configuration according to this embodiment also allows the sensor piece 500 to be properly inserted in the analysis device 105 without depending on the user's vision. In addition, by preparing a plurality of attachments 300 whose projections 320 are of different sizes and have different in-between clearances, it is possible to provide an analysis device 105 of a suitable size for the user's fingers.

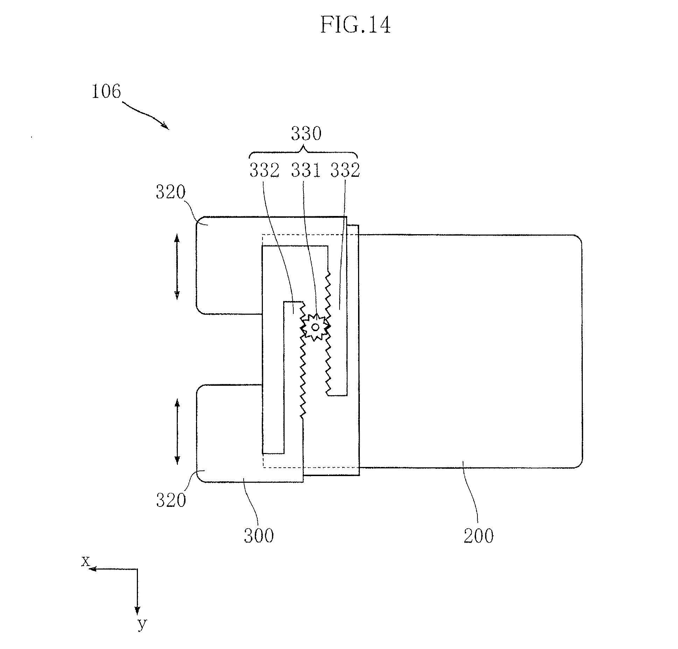

Fig. 14 depicts an analysis device according to a sixth embodiment of the present invention. The analysis device 106 according to this embodiment is different from the analysis device 105 in the configuration of the attachment 300. In this embodiment, the attachment 300 includes a slide mechanism 330. The slide mechanism 330, configured to adjust the in-between clearance of the projections 320 as desired, includes a pinion 331 and a pair of racks 332. Each of the racks 332 is connected to the respective projection 320, and formed so as to extend in the y-direction. The pinion 331 located between the racks 332 so as to be engaged with the both racks 332.

According to this embodiment, the in-between clearance of the projections 320 can be properly adjusted in accordance with the size of the user's fingers. The configuration of the pinion 331 and the racks 332 to constitute the slide mechanism 330 causes the projections 320 to move symmetrically with respect to the center of the main body 200 in the y-direction. Thus, the insertion slot 210 is constantly located at the center between the projections 320. Such an arrangement enables the user to insert the sensor piece 500 into the insertion slot 210 with ease.

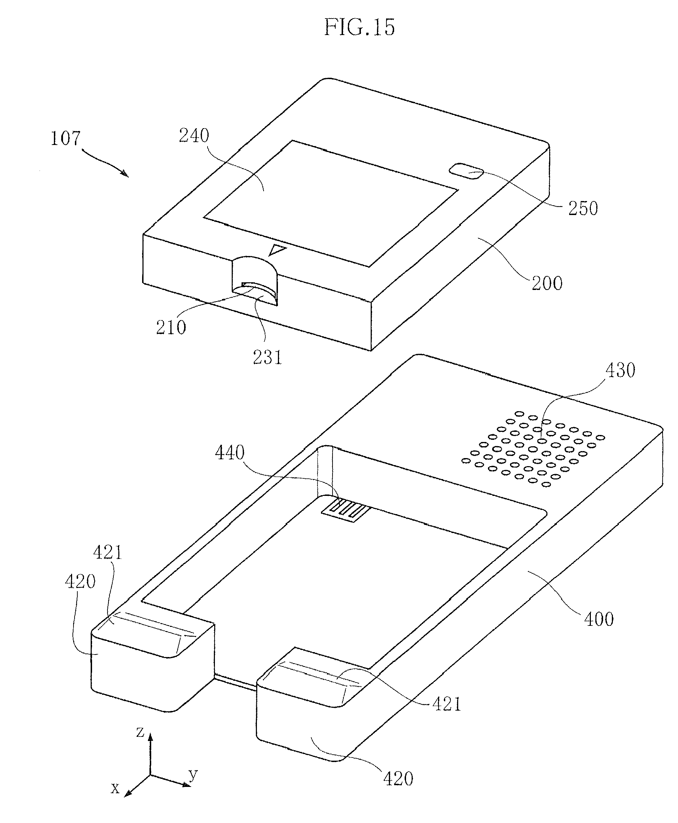

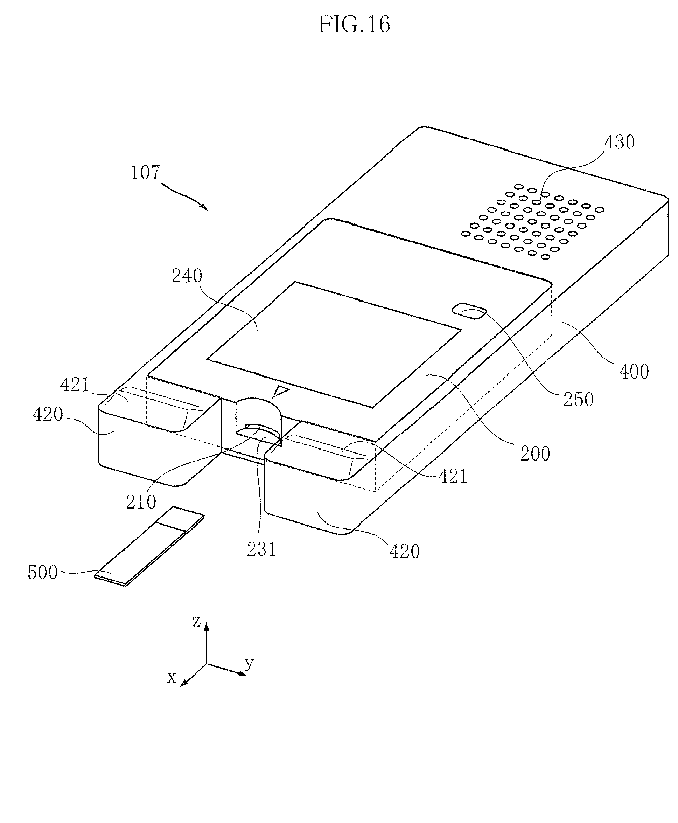

Figs. 15 and 16 depict an analysis device according to a seventh embodiment of the present invention. The analysis device 107 according to this embodiment is different from the foregoing embodiments in including an attachment with audible guidance 400 to be attached to the main body 200.

The attachment with audible guidance 400 can be attached to the main body 200, and includes a pair of projections 420, a speaker 430, and a terminal 440 as shown in Fig. 15. The projections 420 are configured similarly to the projections 320 of the analysis device 104, and includes an elevated portion 421. The terminal 440 serves as an interface with the main body 200, and is located so as to contact the terminal (not shown) of the main body 200. The speaker 430 outputs an audible guide. The attachment with audible guidance 400 includes a control unit, a storage unit, a power source, and so forth, though not shown. When a signal indicating a status of the main body 200 is inputted to the attachment with audible guidance 400 through the terminal 440, the control unit controls the speaker 430 to output an audible message representing the status of the main body 200.

The configuration according to this embodiment also allows the measurement operation to be properly performed, without depending on the user's vision.

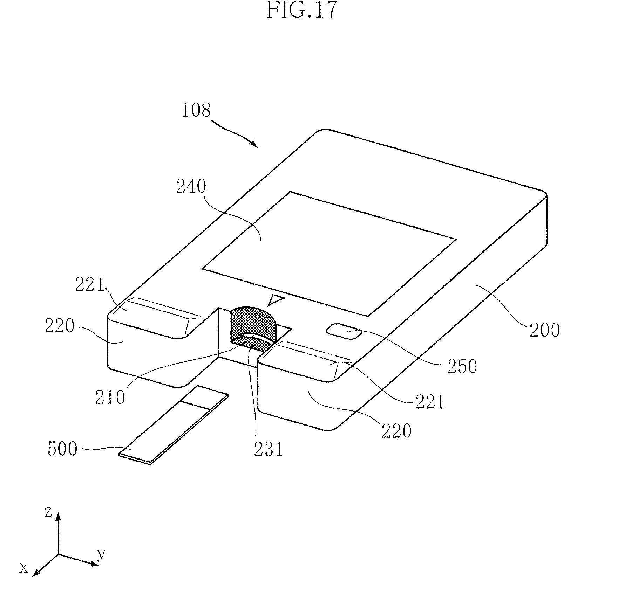

Fig. 17 shows an analysis device according to an eighth embodiment of the present invention. The illustrated analysis device 108 is the same as the above-noted analysis device 101 except that portions around the insertion slot 210 are colored for recognition.

In the analysis device 108, the colored portions include the guide surface 231 adjacent to the insertion slot 210, and a region adjacent to and above the insertion slot 210. As an example, when the analysis device 108 as a whole is white, a color such as red may be adopted, which makes a striking contrast with respect to white. Such a configuration helps a weak-sighted user to readily recognize the position of the insertion slot 210. To enhance this advantageous feature, it is preferable to adopt a complementary color for the partial coloring, with respect to the color provided on the analysis device 108 as a whole. As readily understood, such a coloring can also be applied to the analysis devices 102 to 107.

The analysis device according to the present invention is not limited to the foregoing embodiments. The specific configurations of the respective parts of the analysis device may be modified in various manners.

The analysis device according to the present invention is used not only for a biosensor including a working electrode and a counter electrode, but also for a biosensor configured to analyze a body fluid by using colorimetry.

## Claims

1. An analysis device (101; 102; 103; 104; 105; 106; 107; 108) comprising:
a main body (200);
a sensor piece slot (210) for supporting a sensor piece (500) in a manner such that the sensor piece sticks out from the main body; and
a pair of projections (220; 320; 420) spaced apart from each other in a width direction perpendicular to a sticking direction of the sensor piece, with the sensor piece slot present between the projections, each of the projections protruding outwardly in the sticking direction of the sensor piece and having a height overlapping the sensor piece slot in a height direction perpendicular to both the sticking direction and the width direction.

2. The analysis device according to claim 1, wherein the sensor piece slot (210) is configured for insertion of the sensor piece (500) from outside of the analysis device.

3. The analysis device according to claim 1 or 2, further comprising a guide surface (231) adjacent to the sensor piece slot (210) and extending from the sensor piece slot in the sticking direction.

4. The analysis device according to claim 1, 2 or 3, wherein the sensor piece slot (210) is at a position offset downwardly in the height direction.

5. The analysis device according to any of claims 1-4, further comprising at least a pair of sloped surfaces adjacent to the sensor piece slot (210), with the sensor piece slot present between the sloped surfaces, wherein the sloped surfaces are arranged to become further from each other as proceeding away from the sensor piece slot in the sticking direction.

6. The analysis device according to any one of claims 1-5, wherein each of the projections (220; 320; 420) includes an elevated portion (221; 321; 421) protruding in the height direction.

7. The analysis device according to claim 6, wherein the elevated portion (221; 321; 421) is located outwardly of the sensor piece slot (210) in the sticking direction.

8. The analysis device according to any of claims 1-7, wherein the projections (220; 320; 420) are movable relative to each other in the width direction.

9. The analysis device according to claim 8, wherein the projections (220; 320; 420) move symmetrically with respect to a center of the sensor piece slot (210).

10. The analysis device according to any of claims 1-9, further comprising a resistance applier (261; 262) that applies a resisting force to the sensor piece (500) inserted through the sensor piece slot (210) at a resistance applying position,
wherein, when the sensor piece is in a measurement position, a distance (d1) between a leading end of the respective projections (220; 320; 420) in the sticking direction and a leading end of the sensor piece (500) in the sticking direction is shorter than a distance (d2) between an opposite end of the sensor piece and the resistance applying position.

11. The analysis device according to claim 10, wherein the resistance applier (261; 262) comprises an electrode (261) coming into contact with the sensor piece (500) or a detection lever (262) for detecting presence of the sensor piece (500).

12. The analysis device according to any of claims 1-11, wherein the projections (220; 320; 420) are formed of a material softer than a material of the main body (200).

13. The analysis device according to any of claims 1-12, further comprising an attachment (300; 400) removably attachable to the main body (200), wherein the projections (220; 320; 420) are formed on the attachment.

14. The analysis device according to claim 13, wherein the attachment (400) is provided with an audible guidance function.
